Europäisches Patentamt

European Patent Office

Office européen des brevets

(11) Numéro de publication: **0 148 047 B1**

(12) **FASCICULE DE BREVET EUROPEEN**

(45) Date de publication du fascicule du brevet: **11.05.88**

(21) Numéro de dépôt: **84402370.5**

(22) Date de dépôt: **21.11.84**

(51) Int. Cl.⁴: **A 61 F 5/44**

(54) **Ensemble pour incontinent.**

(30) Priorité: **21.11.83 FR 8318508**

(43) Date de publication de la demande:
**10.07.85 Bulletin 85/28**

(45) Mention de la délivrance du brevet:
**11.05.88 Bulletin 88/19**

(84) Etats contractants désignés:
**AT BE CH DE FR GB IT LI LU NL SE**

(56) Documents cités:
**FR-A-2 477 881**
**FR-A-2 498 447**

(73) Titulaire: **Nigay, Pierre**
**10 rue de Paris**
**F-60200 Compiegne (FR)**

(72) Inventeur: **Nigay, Pierre**
**10 rue de Paris**
**F-60200 Compiegne (FR)**

(74) Mandataire: **Thevenet, Jean-Bruno et al**
**Cabinet BEAU DE LOMENIE 55 rue d'Amsterdam**
**F-75008 Paris (FR)**

Courier Press, Leamington Spa, England.

EP 0 148 047 B1

Description

L'invention concerne les sous-vêtements pour personnes atteinte d'incontinence d'urine.

L'incontinence urinaire concerne aussi bien les hommes que les femmes.

On la rencontre d'une manière générale chez les enfants, de la naissance jusqu'à 3 ans. Passé cet âge, il s'agit souvent d'incontinence nocturne concernant parfois des enfants jusqu'à 14 ans cette dernière étant en régression au cours de la croissance. L'incontinence féminine concerne de nombreuses jeunes femmes, en particulier à la suite d'une grossesse. L'incontinence masculine est plus particulière, après une opération de la prostate. L'incontinence des personnes âgées est due à la diminution des forces musculaires et organiques, voire un dérèglement consécutif à la prise de médicaments. Elle est fréquente chez les personnes alitées.

A ce jour, il existe des fournitures dont le rôle est d'absorber les urines, par capillarité, et de les emmagasiner jusqu'à un point de retention plus ou moins important suivant la texture et la nature des matières premières employées.

On connait aussi par le document FR—A—2498447 une culotte de miction pour femme comportant une zone de puisage à laquelle peut s'adapter un tube souple d'évacuation relié à une poche de retenue au travers d'une pompe. Cette culotte est uniquement destinée à une évacuation volontaire: elle nécessite la commande d'un interrupteur. On imagine mal d'exiger des énurétiques nocturnes qu'ils se réveillent d'abord pour commuter l'interrupteur. De plus, il est clair que cette culotte n'est pas conçue pour que les accessoires (tuyaux, poches) soient portés en permanence par le sujet lui-même: il est même indiqué, p.4, 1.30, qu'en fin d'opération le tuyau d'évacuation est débranché. Cette culotte ne saurait donc répondre aux besoins des incontinents.

On connaît par ailleurs, pour le document FR—A—2477881, un appareil destiné aux grabataires mais non aux incontinents qui désirent mener une vie normale. L'appareil proposé n'est pas autonome: il nécessite le branchement électrique sur le secteur (cf. p.7, 1.30). Il ne permet pas d'être porté en permanence, mais nécessite d'être appliqué au moment voulu (cf. p.2, 1.29 et suivantes), ce qui ne convient pas aux incontinents pour lesquels ce moment n'est connu que quand il est trop tard. Finalement, cette appareil est également conçu pour une évacuation volontaire.

La présente invention a pour objet un ensemble qui rende possible le contrôle du flux d'urines dès son commencement. Cet ensemble doit être simple et peu coûteux, sans risque pour l'utilisateur, entrant dans la catégorie de ce qu'il est convenu d'appeler: "sous-vêtements d'hygiène d'utilité courante". Surtout l'invention vise à présenter un ensemble conçu pour être porté intégralement et en permanence par le sujet, et doté d'un fonctionnement automatique ce qui le rend parfaitement adapté à tous les cas d'incontinence.

L'objet de l'invention consiste en une sorte de culotte, slip, suspensoir dont la partie en contact avec le sexe, lorsqu'il est porté, est équipée d'un système de détection d'humidité et d'un orifice d'évacuation relié à un système de pompage. Plus précisément, la culotte de l'invention, réalisée, au moins pour sa partie basse, en matière imperméable s'adaptant de manière étanche au porteur, comporte en partie basse une zone de puisage à laquelle s'adapte un tube souple d'évacuation relié à une poche de retenue au travers d'une pompe électrique. L'ensemble de l'invention comprend également un support porté par le sujet et retenant d'une part un boîtier logeant la pompe et la source de courant continu basse-tension, et d'autre part la poche de retenue. D'autre part, il est prévu un circuit électrique temporisé de déclenchement de la pompe, alimenté par la source de courant et comportant des moyens de déclenchement réagissant au liquide disposés près de la zone de puisage et reliés par des fils électriques souples audit dispositif.

L'ensemble de l'énergie nécessaire au fonctionnement pour la détection, la commande de mise en route et d'arrêt du système de pompage provient de piles ou batteries.

Il est toutefois particulièrement avantageux que le mécanisme de pompage fasse appel à un appareillage de mise en réserve de la force motrice. Ce dernier peut être constitué par un ressort remontable ou par la mise sous pression par gavage d'un fluide ou d'un gaz aux fins de détente. En effet, la pompe de l'invention doit assurer un débit rapide dans un temps relativement court, et ce à des intervalles de temps relativement long. Il est donc intéressant que l'énergie des batteries soit utilisée pendant ces intervalles pour augmenter la puissance disponible pendant le temps d'utilization, sans surdimensionner la pompe et/ou l'alimentation électrique.

Conformément à l'invention, la culotte peut être réalisée entièrement en matière imperméable ou seulement le fond. Elle peut être doublée partiellement ou entièrement en sa partie intérieure (et, ou extérieure) d'un tissu perméable.

L'aspiration peut concerner l'élimination des urines et des matières fécales liquides.

Les sous-vêtement peut épouser diverses formes pour faciliter l'étanchéité, l'emmagasinage des impuretés et la facilité de leur écoulement. Il peut être utilisé avec ou sans couche.

Le système de détection d'arrivée du liquide est constitué, soit d'électrodes, soit de flotteurs ou de sondes qui transmettent l'information à un appareillage à commande électrique.

Les pompes de type aspirante, foulante, aspirante et foulante, à piston plongeur, centrifuge, rotative à pignon, rotative à palette, péristaltique, à membrane, à vis, à hydro-éjecteurs, auto-amorçante avec clapet ou crépine, électro-pompes submersibles, peuvent convenir. Les pompes silencieuses auto-amorçantes sont préférées.

Le débit de la pompe est avantageusement compris entre 1 et 30 cm³ à la seconde, étant entendu qu'il est de préférence voisin de 20 à 30 cm³.

Le voltage de la force motrice est compris entre 5 et 25 Volts.

L'equipement électrique et/ou électronique (détection) est monté sur un support (tel qu'une ceinture).

L'équipement de pompage et de stockage peut être utilisé sur des culottes interchangeables.

L'installation peut fonctionner alors que le sujet est habillé de sa culotte, le dispositif de pompage et de stockage du liquide étant porté à la ceinture, relié seulement par ses fils électriques de détection et son tuyau d'évacuation.

La transmission du liquide au stockage peut être assurée par un conduit à capillarité, au moyen de corps spongieux absorbants. Ces derniers peuvent être activés par des polarisations ioniques.

L'avantage d'un tel équipement est de recueillir les urines au fur et à mesure de leur émission et de les emmagasiner dans un récipient.

D'autres avantages sont, notamment: la protection des odeurs dans l'environnement de l'incontinent et la suppression du bain de siège imposé par les produits existant actuellement sur le marché, la suppression de fuites incontrôlées d'écoulement par les jambes de la culotte, la suppression de la mouille — par capillarité — des vêtements des parties supérieures et inférieures du sujet.

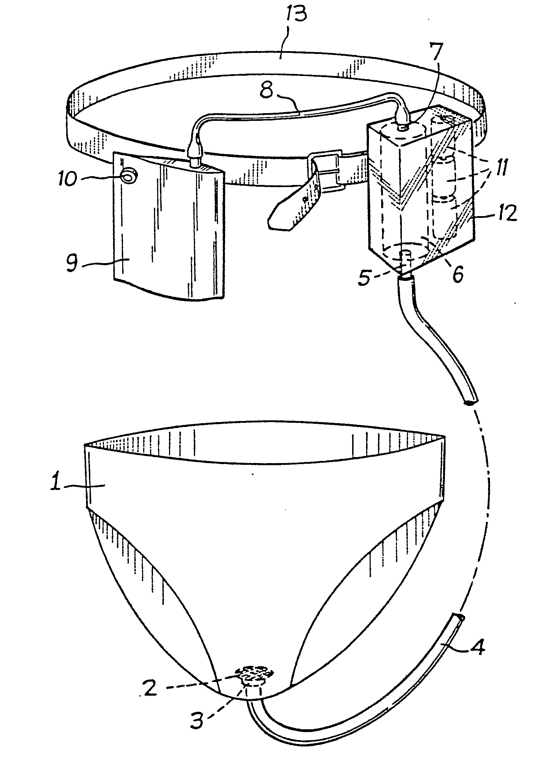

L'invention sera mieux comprise à la lecture de la description suivante d'un mode particulier et non limitatif de réalisation. Il sera fait référence à la figure unique annexée qui représente schématiquement un dispositif conforme à l'invention.

On y voit un slip ou culotte 1 imperméable comportant au fond de l'intrejambe une prise d'aspiration 2, dotée d'un raccord 3 permettant le branchement d'un tuyau souple 4. Le tuyau souple 4 est branché de l'autre côté sur l'entrée d'aspiration 5 d'une pompe 6, dont la sortie de refoulement 7 est raccordée, par un autre tuyau souple 8, à une poche 9 souple de retenue de liquide pourvue d'une valve de décompression 10. Afin que la poche 9 piusse fonctionner dans toutes les positions du porteur, il est prévu à l'intérieur un flotteur portant sur sa partie émergée l'extrémité d'un tuyau suffisamment long et très souple dont l'autre extrémité est reliée à la valve 10. Ainsi, quelle que soit la position de la poche, le flotteur maintient en dehors du liquide l'extrémité du tuyau qui assure la décompression de la poche.

Au voisinage de la prise d'aspiration 2 se trouvent, non représentées, les électrodes de détection du liquide: elles sont reliées, par un câblage électrique courant en parallèle avec le tuyau souple 4, à un circuit électrique de déclenchement (de type conventionnel) des contacteurs de l'alimentation électrique de la pompe 6. Ce circuit comprend avantageusement un élément temporisateur pour l'arrêt de la pompe. La pompe 6, ledit circuit électrique, ainsi que les batteres 11 nécessaires à leur alimentation, sont contenues dans un boîtier 12.

Le boîtier 12 est retenue à un côté de la ceinture 13, tandis que la poche 9 est retenue de l'autre côté pour équilibrer les charges.

## Revendications

1. Ensemble pour incontinent comprenant une culotte (1) réalisée au moins pour sa partie basse en matière imperméable s'adaptant de manière étanche au porteur et comportant en partie basse une zone de puisage (2) à laquelle s'adapte un tube souple (4) d'évacuation relié à une poche de retenue (9) au travers d'une pompe électrique et comprenant une source de courant continu basse-tension, (6), caractérisé en ce que l'ensemble comprend également un support (13) destiné à être porté en permanence par le porteur, ce support (13) retenant d'une part un boîtier (12) logeant la pompe et la source de courant continu basse-tension, et d'autre part la poche de retenue (9) et en ce qu'il est prévu un circuit électrique temporisé de déclenchement de la pompe, alimenté par la source de courant et comportant des moyens de déclenchement réagissant au liquide disposés près de la zone de puisage (2) et reliés par des fils électriques souples audit circuit.

2. Ensemble selon la revendication 1, caractérisé en ce que la pompe (6) est associée à un appareil de mise en réserve de la force motrice.

3. Ensemble selon l'une quelconque des revendications 1 ou 2, caractérisé en ce que le support est une ceinture (13).

4. Ensemble selon la revendication 3, caractérisé en ce que le boîtier (12) et la poche (9) sont placés chacun d'un côté de la ceinture (13) et sont reliés par un tuyau souple (8).

5. Ensemble selon l'une quelconque des revendications 1 à 4, caractérisé en ce que la poche (9) comporte un tuyau souple dont une extrémité est fixée à la partie émergeante d'un flotteur et dont l'autre extrémité est reliée à une valve de décompression (10).

## Patentansprüche

1. Vorrichtung für Inkontinenz, bestehend aus einer Hose (1), die zumindest in ihrem unteren Teil aus einem undurchlässigen Material besteht und die sich dicht an ihren Träger anlegt und in ihrem unteren Teil eine durchlässige Zone (2) aufweist, an die ein flexibler Abzugsschlauch (4) anschließt, der über eine elektrische Pumpe (6), die eine Neiderspannungsgleichstromquelle aufweist, mit einem Sammelbehälter (9) verbunden ist, dadurch gekennzeichnet, daß die Vorrichtung auch eine Tragvorrichtung (13) aufweist, die dazu bestimmt ist, dauernd vom Träger getragen zu werden, wobei auf der Tragvorrichtung eine Schachtel (12), die die Pumpe und die Niederspannungsgleichstromquelle enthält und der Sammelbehälter (9) angebracht ist, und daß weiters ein von der Stromquelle versorgter elektrischer Schaltkreis vorgesehen ist, der die Pumpe zweitweise einschaltet und wobei Mittel vorgesehen sind, die auf die Anwesenheit von Flüssigkeit nahe der durchlässigen Zone (2) reagieren und mit dem Schaltkreis durch flexible Stromleitungen verbunden sind, um die Pumpe in Betrieb zu setzen.

2. Vorrichtung nach Anspruch 1, dadurch gekennzeichnet, daß der Pumpe (6) ein Apparat zur Speicherung der Motorkraft zugeordnet ist.

3. Vorrichtung nach einem der Ansprüche 1 oder 2, dadurch gekennzeichnet, daß die Tragvorrichtung ein Gürtel (13) ist.

4. Vorrichtung nach Anspruch 3, dadurch gekennzeichnet, daß die Schachtel (12) une der Behälter (9) jeweils auf einer Seite des Gürtels (13) angeordnet und durch eine flexible Leitung (8) verbunden sind.

5. Vorrichtung nach einem der Ansprüche 1 bis 4, dadurch gekennzeichnet, daß der Behälter (9) aus einem flexiblen Rohr besteht, das mit einem Ende mit dem Ausgangsteil eines Schwimmers verbunden ist, und dessen anderes Ende mit einem Entlüftungsventil (10) versehen ist.

**Claims**

1. A system for the incontinent comprising a pair of knickers or briefs (1), at least the lower part of which is made of waterproof material fitting the wearer in sealing-tight relationship and having at the lower part a draining zone (2) to which there is fitted a flexible drainage tube (4) connected to a retaining bag (9) via an electrical pump (6), and comprising a low-voltage d.c. supply characterised in that the system also comprises of support (13) adapted to be permanently worn by the wearer, said support (13) carrying a box (12) which houses the pump and the low-voltage d.c. supply and also the retaining bag (9), and in that a pump triggering delay circuit is provided which is fed by the current supply and comprises triggering means responding to the liquid, said means being disposed near the draining zone (2) and being connected to said circuit by flexible leads.

2. A system according to claim 1, characterised in that the pump (6) is connected to a device for storing the drive power.

3. A system according to claim 1 or 2, characterised in the support is a belt (13).

4. A system according to claim 3, characterised in that the box (12) and the bag (9) are each disposed on one side of the belt (13) and are connected by a flexible tube (8).

5. A system according to any one of claims 1 to 4, characterised in that the bag (9) has a flexible pipe, one end of which is fixed to the emergent part of a float and the other end of which is connected to a decompression valve (10).